# EUROPEAN PATENT APPLICATION

(11) **EP 2 161 243 A1**
(43) Date of publication of application: **10.03.2010**
(21) Application number: 08163331.5
(22) Date of filing: 29.08.2008
(51) Int. Cl.: C01B 39/00, B01J 29/04, B01J 49/00, C01B 37/04, C01B 37/06, C01B 39/54

(54) **Method for preparing metalloaluminophosphate (MeAPO) molecular sieves**

(71) Applicant: Total Petrochemicals Research Feluy, 7181 Seneffe (Feluy) (BE)
(72) Inventor: Nesterenko, Nikolai, B-1400, Nivelles (BE); Dath, Jean-Pierre, 7970, BELOEIL HAINAULT (BE); Van Donk, Sander, B-1180, Uccle (BE); Vermeiren, Walter, B -3530, Houthalen (BE)
(74) Representative: Neel, Henry

(57) **Abstract**

The invention relates to a process for obtaining a metalloaluminophosphate (MeAPO) molecular sieve comprising the following steps in the order given:
a) providing an aqueous solution containing sources of at least 2 of the following:
- aluminium (Al),
- phosphorous (P),
- metal (Me);

b) adding a first MeAPO molecular sieve to the solution and modifying the pH before and/or after the addition of the first MeAPO molecular sieve to obtain an amorphous precursor;
c) separating the amorphous precursor from the water;
d) optionally washing and drying at a temperature below 450°C of the amorphous precursor;
e) contacting the amorphous precursor with an organic template-containing aqueous solution and with a source of Al, P or Me, which is not already present in step (a), optionally additional sources of Al and/or P and/or Me and optionally in the presence of aliphatic alcohols;
f) performing a crystallization of the amorphous precursor under autogeneous conditions so as to increase the concentration of the crystalline molecular sieve in respect to the initial precursor and so as to obtain a second MeAPO molecular sieve.

## Description

### [Field of the invention]

The present invention relates to a method for preparing metalloaluminophosphate (MeAPO) molecular sieve by pseudo-dry synthesis re-using spent or using fresh molecular sieves. The metalloaluminophosphate molecular sieves of the invention are useful as catalysts in a variety of processes including cracking, hydrocracking, isomerization, reforming, dewaxing, alkylation, transalkylation, conversion of oxygenates to light olefins.

### [Background of the invention]

The limited supply and increasing cost of crude oil has prompted the search for alternative processes for producing hydrocarbon products. One such process is the conversion of oxygen-containing (by way of example methanol), halogenide-containing or sulphur-containing organic compounds to hydrocarbons and especially light olefins (by light olefins it is meant C₂ to C₄ olefins) or gasoline and aromatics. In the present application the conversion of said oxygen-containing (also referred to as oxygenates), halogenide-containing or sulphur-containing organic compounds to hydrocarbons and especially light olefins is referred to as the XTO process. The interest in the XTO process is based on the fact that feedstocks, especially methanol can be obtained from coal, biomass, organic waste or natural gas by the production of synthesis gas, which is then processed to produce methanol. The XTO process can be combined with an OCP (olefins cracking process) process to increase production of olefins. The XTO process produces light olefins such as ethylene and propylene as well as heavy hydrocarbons such as butenes and above. These heavy hydrocarbons are cracked in an OCP process to give mainly ethylene and propylene.

The XTO process can be carried out, for example, with MeAPO molecular sieve catalysts. The procedures for synthesizing metalloalumophophates results in crystalline materials that are often less than a few microns in size. Such fine powders are difficult to use in many industrial processes. For easy handling, the materials have to be formulated with acceptable granulometric and mechanical properties. On an industrial scale, the molecular sieve is generally combined with other materials that densify and provide additional hardness to the finished catalyst product and increase the size of the formulated particles, thus improving the crush strength or attrition resistance of the catalyst under industrial operating conditions. These materials can be various inert or catalytically active matrix materials and/or various binder materials. Some binder materials can also serve as diluents in order to control the rate of conversion from feed to products and consequently improve selectivity.

However, combining the catalyst with matrix materials and/or binder entails an additional step in the catalyst production process. Preparation of the formulated catalyst with elevated content of active phase (MeAPO), which meet the granulometric requirement and particle hardness is difficult. The recipes for the catalyst shaping are very empirical. Methods for shaping the materials include extruding, agglomeration, spray drying and the like, thereby increasing the complexity of catalyst manufacture and provoking high losses of MeAPO molecular sieve. These additional steps may also have an adverse effect on the catalyst performance. Thus it would be advantageous to develop a MeAPO crystalline molecular sieve, which does not require any additional binder material or at least reduces the amount of required binder in industrial applications, but which is yet hydrothermally stable under XTO conditions and can selectively produce light olefins, such as ethylene and/or propylene, from oxygen-containing (also referred to as oxygenates), halogenide-containing or sulphur-containing feedstocks.

Organic template is the most expensive component used for synthesising MeAPO molecular sieves. It is the main determinant for the price of the final product. A lot of effort has been directed to reducing the amount of template used and to replace the more expensive molecule with less expensive alternatives. However, the use of less expensive molecules requires higher molar ratio template/P₂O₅. Thus, the use of templating agent to form crystalline molecular sieves is also problematic and costly. When producing molecular sieves it would be advantageous to also reduce the amount of required templating agent.

A significant amount of MeAPO is also wasted during use for its specified purpose e.g. in an XTO process, due to losses because of attrition in the process and due to irreversible deactivation in the process. During formulation of MeAPO sieves, as in agglomeration and spray-drying, often a lot of fines particles not suitable for industrial use, are produced. It would be interesting if these spent MeAPO and especially the attrition particles (fines) thereof could be re-used. Likewise, it would be advantageous to find a use for the unused unsuitable fines produced during formulation. It would be particularly interesting to find a method, which can recycle spent MeAPO and in particular spent MeAPO's attrition particles (fines), but also implement unused fresh fines normally unsuitable for industrial use.

The prior arts below illustrate different procedures for MeAPO particles recycling or rejuvenating.

US 7,358,412 discloses a method of making a molecular sieve catalyst. The method discloses a way to increase the yield of the good fraction of a spray-dried molecular sieve. The attrition fines are re-used in spray-drying.

EP 1 301 274 discloses a synthesis of a molecular sieve catalyst wherein the attrition particles are recycled in spray-drying with fresh molecular sieve and binders.

US2008/0015402 A1 discloses a method for rejuvenating deactivated molecular sieve. This invention is directed to a method of rejuvenating a molecular sieve that has lost catalytic activity as a result of contact with moisture, and a method of using the rejuvenated catalyst to make an olefin product from methanol feed. The molecular sieve can be rejuvenated by heating at a rate sufficient to increase the catalytic activity of the molecular sieve.

US 2007/0004951 discloses a method for recovering the activity of a molecular sieve particle. The invention is directed to a method of rejuvenating silicoaluminophosphate molecular sieve catalysts that have been deactivated hydrothermally. It also discloses a method of using the rejuvenated catalyst to make an olefin product from an oxygenate feed. In particular, the invention is directed to rejuvenating the catalyst by contacting it with warm water, ammonium salts, dilute acids or low pressure steam until the catalytic activity level of the catalyst has been increased to the desired extent.

These prior art disclosures are different from the Applicant's invention, because they do not disclose the recrystallisation of a precursor in the presence of small amounts of MeAPO, which can be spent/deactivated MeAPO obtained from a XTO or OCP reactor. This difference leads to a more efficient re-use of spent/deactivated MeAPO, especially attrition particles (fines) of spent/deactivated MeAPO. The resulting MeAPO molecular sieve possesses a very small crystal size and is therefore notably more specific for propylene. It should also be noted that the re-use of spent/deactivated MeAPO significantly reduces the need of expensive templating agent.

WO 03/040037 concerns a method for the production of microporous crystalline metalloaluminiumphosphates for use as an adsorbent or a catalyst by at least partially filling the pores of particles containing aluminium phosphate (AIPO) with an aqueous mixture containing any active source of the metal and an organic structure directing agent and performing crystallisation at elevated temperature under autogeneous pressure to obtain crystals of the metalloaluminophosphate. The document discloses that particles may be prepared from the mixture of the crystallised material and a suitable binder in order for it to be suitable for use in industrial MTO processes.

WO 08/019586 relates to a process for preparing microspherical catalyst containing SAPO molecular sieve. The present invention also relates to a catalytic use of the catalyst in a reaction for converting oxygen-containing compound into low-carbon olefins. The document discloses an *in situ* synthesis method of microspherical catalyst used for converting oxygen-containing compound into olefin, characterized in that, firstly preparing microsphere containing silicon phosphor aluminum oxide by spray-drying process; then *in situ* forming SAPO molecular sieve within the microsphere and on its surface by hydrothermal synthesis process. Complete transformation of the precursor to the MeAPO is envisaged.

WO 03/101892 concerns a method for the production of microporous crystalline metalloaluminiumphosphates based adsorbents or catalysts by *in-situ* crystallisation of the metalloaluminiumphosphate inside a formed body. The formed body is prepared from AIPO and binder. Thereafter, the organic structure directing agent (i.e. template), metal source and water are added. The metalloaluminiumphosphate is then crystallised in-situ at elevated temperature and pressure, whilst maintaining the shape and size of the formed body. This divulgation teaches that additional binder is required in the original formed body i.e. in the formulated AIPO, before adding other components and crystallising. Formulation takes place prior to crystallisation.

US 4,861,743 discloses a non-zeolitic molecular sieve prepared by contacting a precursor body of alumina or silica-alumina (optionally containing reactive sources of phosphorus pentoxide and/or other elements desired in the non-zeolitic molecular sieve) with a liquid reaction mixture containing a reactive source of phosphorus pentoxide (and optionally reactive sources of silica and/or other elements desired in the non-zeolitic molecular sieve), and an organic templating agent, thereby causing the body to react with the liquid reaction mixture and to form crystals of the non-zeolitic molecular sieve within the body. The precursor body is made from a dry mix of the Al source and optional Si source, which is extruded as a paste into amorphous shaped bodies, which are preferably calcined prior to the addition of the liquid reaction mixture containing the P source. This document teaches that the phosphorous has to be added after formulation. The mixture will not be particularly homogeneous.

WO 94/13584 relates to the preparation of aluminosilicate zeolites from a reaction mixture containing an amount of water sufficient so that the reaction mixture may be shaped. In the method, the reaction mixture is heated at crystallization conditions and in the absence of an external liquid phase, so that excess liquid need not be removed from the crystallised material prior to drying the crystals. The template is added simultaneously with the silica and alumina sources. This would lead to complete crystallisation of the shaped precursor.

US 5,514,362 discloses a process for the preparation of a non-zeolitic molecular sieve from a dense gel containing sufficient liquid that the dense gel may be formed into self-supporting particles prior to crystallization. In the process, the dense gel, which is optionally in the form of particles, is heated at crystallization conditions in the absence of an external liquid phase, so that excess liquid need not be removed at the conclusion of the crystallization step. This gel comprises the Al source, P source and the templating agent simultaneously. A Si source may be optionally included in the dense gel. By including the templating agent in the formation of the dense gel, complete crystallisation is envisaged.

US2005/0063901 discloses molecular sieves prepared by forming an aqueous reaction mixture slurry comprising an active source of silicon oxide and an organic templating agent, spray drying the reaction mixture slurry to form particles, and heating the spray dried reaction mixture at a temperature and pressure sufficient to cause crystallization of the molecular sieve. The template may in addition also be added to the formed particles after spray drying. In particular, it is alleged that adding all the template to the spray dried material prior to heating may result in no crystallization. The molecular sieves disclosed in this document do not comprise any P, since it only relates to zeolitic molecular sieves. MeAPO are not envisaged.

The invention thus aims to overcome at least one of the problems of the prior art cited above.

It is an aim of the invention to develop a MeAPO molecular sieve that is easily manufactured.

It is further an aim of the invention to recycle/rejuvenate spent/deactivated MeAPO.

It is further an aim of the invention to recycle the attrition particles of molecular sieves after use.

It is also an aim of the invention to develop new MeAPO molecular sieves from solutions of metal (Me), Al and P sources.

It is additionally an aim of the invention to prepare new MeAPO molecular sieves using a minimum amount of aqueous medium during the crystallisation step.

It is further an aim of the invention to provide a method for preparing MeAPO molecular sieves using reduced amounts of the templating agent.

### [Brief Summary of the invention]

The invention relates to a method for preparing molecular sieves wherein the presence of a small amount of a first crystalline MeAPO, which is either fresh or spent, in the amorphous precursor allows converting said precursor to a second MeAPO molecular sieve using a lower amount of organic template than previously necessary. The second molecular sieve can be the same or different.
Thus the invention also covers recycling MeAPO attrition particles (fines) and/or recycling/rejuvenating spent/deactivated MeAPO and/or re-using the waste catalyst. It also covers the use of fresh molecular sieves to be mixed with the amorphous precursor.
This is performed by means of incorporation of the said previously synthesized first MeAPO molecular sieve into the amorphous precursor followed by template addition and crystallization. This is carried out by partial or complete crystallization of the amorphous precursor with the first MeAPO leading to the formation of a higher amount of crystalline phase of MeAPO into the amorphous solid than was added before crystallization. The amorphous precursor can be very easily formulated before or after partial crystallization.
The invention thus covers a process for obtaining a MeAPO molecular sieve catalyst comprising the following steps in the order given:
a) providing a solution containing sources of at least 2 of the following:
   aluminium (Al),
   phosphorous (P),
   metal (Me);
b) adding a first MeAPO molecular sieve to the solution and modifying the pH before and/or after the addition of the first MeAPO molecular sieve to obtain an amorphous precursor;
c) separating the amorphous precursor from the water, optionally including formulation;
d) optionally washing and drying at a temperature below 450°C of the amorphous precursor;
e) contacting the amorphous precursor with an organic template-containing aqueous solution and with a source of Al, P or Me, which is not already present in step (a), optionally additional sources of Al and/or P and/or Me and optionally in the presence of aliphatic alcohols;
f) performing a crystallization of the amorphous precursor under autogeneous conditions so as to increase the concentration of the crystalline molecular sieve in respect to the initial precursor and obtain a second MeAPO molecular sieve.

The molecular sieve formed in step f) could be the same or different from that added during step a).

The first MeAPO molecular sieve can be a calcined and/or non-calcined and/or spent MeAPO molecular sieve. The definition of the first MeAPO molecular sieve also includes unspent MeAPO attrition fines (calcined or non-calcined) recovered from the formulation of a previous MeAPO molecular sieve synthesis.

The amount of calcined, non-calcined or spent MeAPO molecular sieve or MeAPO attrition particles added during step (b) can be varied in a large range, preferably at least 0.1 wt%, more preferably at least 1 wt% and most preferably from 2 to 50wt%. The weight percentage being defined in respect of the dry composition (Al₂O₃/P₂O₅/SiO₂) of the mixture prepared in step (a).

Additional binders & fillers can be added to the solution of any one of steps (a) or (b).

In the cases where the co-precipitated amorphous precursor is not formulated during step (c), the process may also comprise a further step (g) wherein the molecular sieve obtained from step (f) is formulated by extrusion or spray-drying, optionally in the presence of other compounds.

The process may also comprise a further step (h) wherein the molecular sieve obtained from step (f) or step (g) is calcined, steamed or ion-exchanged.

Preferably, the solution obtained at step (a) has a pH lower than 4 or higher than 8. Preferably, the pH is changed in step (b) to a pH of from 4 to 8. The pH can be changed to a pH of 4 to 8 before addition of the first MeAPO molecular sieve to obtain the amorphous precursor. It can then be changed again to increase precipitation of the precursor. However, it should preferably remain within a pH of between 4 and 8. Alternatively, the pH is only changed to a pH of 4 to 8 after addition of the first MeAPO molecular sieve to obtain the amorphous precursor.

The invention further covers the MeAPO molecular sieve obtained by any one of the above processes.

The invention also covers the use of such MeAPO molecular sieves in XTO processes and XTO/OCP combined processes.

The invention also covers the process of recycling MeAPO during an XTO process and XTO/OCP combined process.

### [Brief description of the drawings]

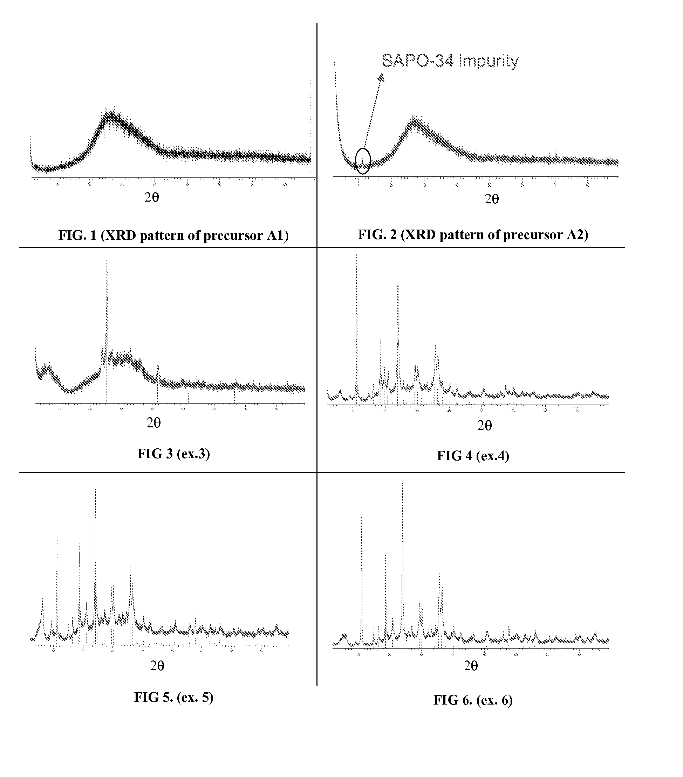

Figures 1-6 represents XRD patterns of various MeAPO-containing catalysts obtained from an amorphous precursor.

### [Detailed description of the invention]

Accordingly, this invention provides an improved process with respect to cost and efficiency for obtaining crystalline MeAPO molecular sieves from an amorphous solid precursor. The essence of the invention is provided by the use of a dense amorphous solid precursor containing a small amount of MeAPO for manufacturing a catalyst with higher MeAPO molecular sieves content, obtained from an aqueous solution comprising sources of at least 2 of the following: Al, P and Me and molecular sieve. Such sources are cost-efficient and readily available. Molecular sieve could be subjected to milling to reduce the particle size to less than 4 µm before adding to the solution. This method allows significantly reducing the amount of template used to crystallize the MeAPO from amorphous precursor because the crystallization can be performed under milder conditions in the presence of crystalline phase.

The invention also proposes a solution to recycle the attrition particles from the XTO process unit, recycle/rejuvenate spent/deactivated MeAPO, re-use the waste MeAPO catalyst. Fresh un-used catalyst can also be used, in particular the attrition particles obtained from the synthesis of a previous MeAPO molecular sieve, since another aspect of the invention is the reduction in the required amount of templating agent.

The use of the amorphous solid allows a reduction in the amount of water and the crystallization step can be done in a very concentrated suspension.

### With regards to step (a), a solution is provided containing sources of at least 2 of the following: Al, P and Me.

The starting solution, from which the homogeneous amorphous precursor is obtained, comprises sources providing either:
i. Al, P, and Me;
ii. Al and P;
iii. Al and Me; or
iv. P and Me;
In combination with a calcined/non-calcined or spent/deactivated MEAPO, whereby embodiments (i) and (ii) are generally preferred. More preferably, embodiment (ii) is preferred.

In the embodiment according to (i), the solution has a molar ratio between the components Al:P & Al:Me of normally from 0,5 to 5 and from 0.2 to 5 respectively, more preferably from 1 to 3 and from 0.25 to 4 respectively.
In the embodiment according to (ii), the solution has a molar ratio between the components Al and P of normally from 0.5 to 5, more preferably of from 1 to 3.
In the embodiment according to (iii), the solution has a molar ratio between the components Al and Me of normally from 0.2 to 100, more preferably of from 0.25 to 5, most preferably from 0.25 to 4.
In the embodiment according to (iv), the solution has a molar ratio between the components P and Me of normally from 0.05 to 15, more preferably of from 0.15 to 10.

In the embodiments, according to (ii), (iii) and (iv), the third component of Al, P and Me not added during step (a) is added together with the templating agent during step (d). Some silicon and phosphorous can be added together with template in spite of the presence of these elements in the initial amorphous solid, such that in the final molecular sieve the molar ratios of P/template is at most 10, preferably at most 4.

It is not necessary that each individual source of Al, P and Me present in step (a) be soluble in water. However, the formed slurry of these components must be stirred continuously to form a homogeneous mixture before proceeding to the next step.

A homogeneous solution of the components is obtained by dispersing and/or dissolving the sources of the individual components in an aqueous medium using a minimum amount of water.

If desired, in the embodiments where Al is present in step (a), i.e. embodiments (i), (ii) and (iii), no additional water need be added at all. The water already present as the water of hydration of the Al-containing source may be sufficient. Alternatively, up to 40 wt % of additional water may be added to the solution, based on the weight of the Al-containing source. More preferably, the solution may contain up to 20 wt % of additional water. Most preferably, the solution may contain up to 5 wt % of additional water. Water may also be added during step (b), as described below, in the form of a basic solution.

**With regards to the sources of Al**, it can be any aluminum species capable of being dispersed or dissolved in an aqueous solution of phosphoric acid. Useful sources of alumina are one or more sources selected from the group consisting of the following: Al(NO3)3, hydrated alumina, peptized alumina, organo aluminium compound, in particularly Al(OiPr)₃, pseudo-boehmite, aluminum hydroxide, colloidal alumina, aluminium halides, aluminium carboxylates, aluminium sulfates, NaAlO₂ and mixtures thereof.

**With regards to the sources of P,** it can be one or more sources selected from the group consisting of phosphoric acid; organic phosphate salts, such as alkali phosphates, in particular triethyl phosphate; ammonium salts such as monobasic ammonium phosphate, dibasic ammonium phosphate and tetraalkyl-ammonium phosphate; aluminophosphates; and mixtures thereof.

**With regards to the source of Me**, it is advantageously one or more metals selected from the group consisting of silicon, germanium, magnesium, zinc, iron, strontium, cobalt, nickel, manganese and chromium. If only Al and P sources are added in step (a), then the one or more metals are added during step (d). Preferred metals are silicon, germanium, magnesium and cobalt with silicon or germanium being especially preferred. Non-limiting examples of useful inorganic silicon sources capable of being dispersed or dissolved in an aqueous solution include colloidal silica, pyrogenic silica (fumed silica), silica sol, metal silicates, precipitated silica, kaolin, organo silicon compounds (like tetraethyl orthosilicate) and silica gel or a mixture of thereof. The metal silicate can be an alkaline earth metal comprising one or more alkaline earth metals selected from Ca, Mg, Sr and Ba. The metal silicates may also comprise other elements selected from one or more of the following: B, Ga, Al, Ce, In, Cs, Sc, Sn, Li, Zn, Co, Mo, Mn, Ni, Fe, Cu, Cr, Ti, La and V. Preferably, the other element is selected from one or more of Al, Mg, Ce, Mg, Co and Zn or mixtures thereof.

The preferred Me source is a calcium silicate with a very open and accessible pore structure. An even more preferred Me source comprises a synthetic crystalline hydrated calcium silicate having a chemical composition of Ca₆Si₆O₁₇(OH)₂ which corresponds to the known mineral xonotlite. Generally, a synthetic hydrated calcium silicate is synthesized hydrothermally under autogeneous pressure. A particularly preferred synthetic hydrated calcium silicate is available commercially from the company Promat of Ratingen in Germany under the trade name Promaxon.

**With regards to step (b)**, the first MeAPO molecular sieve may be added to the amorphous precursor solution. The first MeAPO molecular sieve can be a calcined and/or non-calcined and/or spent MeAPO molecular sieve. The definition of the first MeAPO molecular sieve also includes unspent MeAPO attrition fines (calcined or non-calcined) recovered from the formulation of a previous MeAPO molecular sieve synthesis. The MeAPO molecular sieve may contain template, can be in calcined or dried form, or it can be bound with other compounds (binders, fillers etc). It is not necessary to treat the molecular sieves before adding them to the precursor solution. However, it is preferred that they are milled to reduce the size of the particles to less than 4 µm.

Regarding the content of the MeAPO molecular sieve in the amorphous precursor, the content of MeAPO in the amorphous precursor can be varied in a very wide range. Advantageously, the MeAPO content is at least 0.1 wt% in respect to dry basis of the sum of Al₂O₃+P₂O₅+SiO₂, more preferably is at least 1 wt%, the most preferably is from 2 to 50 wt%.

The amorphous precursor is obtained in step (b) by changing the solution's pH, which can be changed before and/or after addition of the first MeAPO molecular sieve. Preferably, the pH is modified to obtain a pH of 4 to 8.
The pH can be changed, preferably to a pH of 4 to 8, before addition of the first MeAPO molecular sieve in order to obtain the amorphous precursor. After addition of the molecular sieve, it is then possible to change the pH again to increase precipitation of the precursor. However, it should preferably remain within a pH of between 4 and 8.
Alternatively, the first MeAPO molecular sieve is added to the solution without prior modification of the pH. The amorphous precursor is then obtained by modifying the pH, preferably to a pH of 4 to 8, after addition of the molecular sieve.
The change in pH is generally carried out by either adding the acidic phase to the basic phase, or vice versa. One suitable method is to drip or spray or otherwise slowly introduce the acidic phase into the base phase, which results in the production of small spheres or balls once the solution is brought into contact with a large excess of a base. These spheres can then be subsequently collected. If a base is required to increase the pH of the precursor, it is preferably selected from one of ammonium hydroxide, organic amines, alkali or alkali metal salts. Preferably the base is ammonium hydroxide NH₄OH. If an acid is required to decrease the pH of the precursor, it is preferably an inorganic acid. The inorganic acids may comprise an inorganic acid such as nitric acid, hydrochloric acid, methane sulfuric acid, sulfuric acid, phosphoric, carbonic or a salt of such an acid (e.g. the sodium or ammonium salts) or a mixture of two or more of such acids or salts. More preferably the acid is phosphoric acid.

**With regards to step (c)**, the amorphous precursor is then separated from the aqueous medium, optionally including formulation.
In one embodiment, the precipitated amorphous precursor is separated from the water by filtration and/or centrifugation and/or evaporation and/or drying. The evaporation or drying can be performed by heating the solution to a temperature of at least 50°C under atmospheric pressure or under vacuum in a drying unit to form a dried amorphous precursor. The separated amorphous precursor can then be optionally formulated (shaped). This can be carried out by extrusion and/or pelletisation and/or spray drying. Pelletising or extruding are preferred methods for formulation from the separated precursor. If the catalyst is not formulated at this stage, it is preferably formulated at a later stage i.e. step (g). All techniques known to the person skilled in the art are possible. The preferred technique will depend on the selected sources of Al, P and Me and the desired catalyst applications.

In another embodiment, the catalyst is directly formulated (shaped) from the slurry comprising the precipitated precursor e.g. by spray drying or any other way known to the person skilled in the art. The slurry containing the sources of Al, P, Me and the precipitated amorphous precursor is fed to a forming unit that produces a dried formulated amorphous precursor. Non-limiting examples of forming units include spray dryers, pelletizers, extruders, etc. In a preferred embodiment, the forming unit is spray dryer. Typically, the forming unit is maintained at a temperature sufficient to remove most of the liquid (e.g. water) from the slurry.
Optionally, when a spray dryer is used as the forming unit, typically the mixture containing the appropriate sources of Al and/or P and/or Me, is co-fed to the drying unit with a drying gas. In one embodiment the drying unit has an average inlet temperature ranging from 150° C to 550° C, and an average outlet temperature ranging from 100° C to about 250° C.
In one embodiment, the slurry is passed through a nozzle distributing the slurry into small droplets, resembling an aerosol spray, into a drying chamber. Atomization is achieved by forcing the slurry through a single nozzle or multiple nozzles with a pressure drop in the range of from 100 psia to 1000 psia (690 kPa-a to 6895 kPa-a). In another embodiment, the slurry is co-fed through a single nozzle or multiple nozzles along with an atomization fluid such as air, steam, flue gas, or any other suitable gas. In case of the use of multiple nozzles, the co-feeding of another stream containing Si compound is possible.
In yet another embodiment, the slurry described above is directed to the perimeter of a spinning wheel that distributes the slurry into small droplets, the size of which is controlled by many factors including slurry viscosity, surface tension, flow rate, pressure, and temperature of the slurry, the shape and dimension of the nozzle(s), or the spinning rate of the wheel. These droplets are then dried in a co-current or counter-current flow of air passing through a spray drier to form a partially, substantially or totally dried molecular sieve catalyst. An example of a spray drying process that may be used to dry the slurry is disclosed in U.S. Pat. No. 4,946,814, the description of which is incorporated herein by reference.
Optionally, any of the above embodiments comprising formulation can be performed in the presence of various other materials, such as Tylose, matrix materials, various inert or catalytically active materials, or various binder materials, to facilitate and to increase the catalyst's resistance yet further to the temperatures and other conditions employed in the organic conversion processes.

**With regards to optional step (d), for washing and drying** the usual means known to the person skilled in the art can be used. The amorphous precursor of the molecular sieve obtained from step (c) is washed and dried.
In a particular embodiment, the precursor can be washed with water, then washed with a volatile, oxygen-containing water-miscible organic solvent having a relatively low surface tension.
After washing, the molecular sieve precursor is then dried. The obtained precursor must be in an amorphous form. It has been found that the presence of any crystalline phase, different from the added MeAPO of step (c) or desired MeAPO prior to the crystallisation step, may hinder the formation of the desired crystals. Hence, in order to ensure that the solid remains amorphous, it may be calcined prior to step (d). Not only does the calcination ensure that the precursor remains amorphous, but it also changes the reactivity and accessibility of the reactive sites, and hence accelerates the reaction of the precursor with the template-containing solution in step (d). However, the temperature of calcination must be maintained well below the thermal crystallisation temperature of the amorphous precursor.

An acceptable calcination environment is air that typically includes a small amount of water vapour. Typical calcination temperatures are below 450° C, preferably in a calcination environment such as air, nitrogen, helium, flue gas (combustion product lean in oxygen), or any combination thereof.

The dried or formulated molecular sieve catalyst can be calcined in many types of devices, including but not limited to, rotary calciners, fluid bed calciners, batch ovens, and the like. Calcination time is typically dependent on the degree of hardening of the molecular sieve catalyst and the temperature.

In a preferred embodiment, the molecular sieve catalyst is heated in air or in nitrogen at a temperature of from about 100° C. to about 450° C. Heating is carried out for a period of time typically from 30 minutes to 15 hours, preferably from 1 hour to about 10 hours, more preferably from about 1 hour to about 5 hours, and most preferably from about 2 hours to about 4 hours.

**With regards to step (e)**, the amorphous precursor is contacted with a template-containing solution and with a source of Al, P or Me, if not already present in step (a). It is preferred, that the mixture of the precursor and the templating agent-containing solution and, as the case may be, Al, P or Me sources, be stirred with the precursor until the reaction mixture becomes substantially homogeneous.

Additional Al, P and/or Me can be added during step (e) even if already present in step (a). Furthermore, the additional sources of these elements can be the same or different from the ones provided in the solution of step (a).

Advantageously, the reaction mixture at step d) contains a molar ratio between the components P/template of less than 3 and/or Al/P of from 1 to 5 and/or Al/Si from 0.2 to 100, preferably an Al/Si from 0.2 to 5, preferably an Al/Si from 0.2 to 4.

Optionally, aliphatic alcohols can be present in the solution. This aliphatic alcohol acts as a texture influencing agent (TIA). It is not particularly limited. However, it is preferably selected from among 1,2-propanediol, 1,3-propanediol, methanol, ethanol, propanol, isopropanol, butanol, glycerol or ethylene glycol. Preferably, the aliphatic alcohol is ethanol, methanol or ethylene glycol.

**With regards to the templating agent**, it can be any of those heretofore proposed for use in the synthesis of conventional zeolitic aluminosilicates and microporous aluminophosphates. In general these compounds contain elements of Group Va of the Periodic Table of Elements, particularly nitrogen, phosphorus, arsenic and antimony, preferably N or P and most preferably N, which compounds also contain at least one alkyl or aryl group having from 1 to 8 carbon atoms. Particularly preferred nitrogen-containing compounds for use as templating agents are the amines and quaternary ammonium compounds, the latter being represented generally by the formula R₄N⁺ wherein each R is an alkyl or aryl group containing from 1 to 8 carbon atoms. Polymeric quaternary ammonium salts such as [(C₁₄H₃₂N₂)(OH)₂]ₓ wherein "x" has a value of at least 2 are also suitably employed. Both mono-, di- and tri-amines are advantageously utilized, either alone or in combination with a quaternary ammonium compound or other templating compound. Representative templating agents include tetramethylammonium, tetraethylammonium, tetrapropylammonium or tetrabutylammonium cations; di-n-propylamine, tripropylamine, triethylamine; diethylamine, triethanolamine; piperidine; morpholine; cyclohexylamine; 2-methylpyridine; N,N-dimethylbenzylamine; N,N-diethylethanolamine; dicyclohexylamine; N,N-dimethylethanolamine; choline; N,N'-dimethylpiperazine; 1,4-diazabicyclo(2,2,2)octane; N-methyldiethanolamine, N-methylethanolamine; N-methylpiperidine; 3-methylpiperidine; N-methylcyclohexylamine; 3-methylpyridine; 4-methylpyridine; quinuclidine; N,N'-dimethyl-1,4-diazabicyclo(2,2,2)octane ion; di-n-butylamine, neopentylamine; di-n-pentylamine; isopropylamine; t-butylamine; ethylenediamine; pyrrolidine; and 2-imidazolidone. Advantageously organic templating agent is selected among tetraethylammonium hydroxide (TEAOH), diisopropylethylamine (DPEA), tetraethyl ammonium salts, cyclopentylamine, aminomethyl cyclohexane, piperidine, triethylamine, diethylamine, cyclohexylamine, triethyl hydroxyethylamine, morpholine, dipropylamine, pyridine, isopropylamine di-n-propylamine, tetra-n-butylammonium hydroxide, diisopropylamine, di-n-propylamine, n- butylethylamine, din-butylamine, and di-n-pentylamine and combinations thereof. Preferably, the template is a tetraethyl ammonium compound selected from the group of tetraethyl ammonium hydroxide (TEAOH), tetraethyl ammonium phosphate, tetraethyl ammonium fluoride, tetraethyl ammonium bromide, tetraethyl ammonium chloride, tetraethyl ammonium acetate. Most preferably, the template is tetraethyl ammonium hydroxide. These can be added in the form of an aqueous solution.

**With regards to step (f)**, the molecular sieve can be partially or completely crystallised from the suspension or precursor. Preferably, it is crystallised from the suspension or precursor under autogeneous conditions so that 5 to 100% by weight of the amorphous precursor crystallises. Preferably, 5 to 90% by weight of the amorphous precursor is crystallised. More preferably, 5 to 80% by weight crystallises. Most preferably, 5 to 50% by weight crystallises. The autogeneous conditions for crystallisation required here for are well-known in the art. Partial crystallisation is advantageous, because the remaining amorphous phase can act as a binder, thereby reducing or even eliminating the amount of required binder for industrial-scale processes.

The reaction mixture is heated up to the crystallization temperature that may range from about 120°C to 250°C, preferably from 130°C to 225°C, most preferably from 150°C to 200°C. Heating up to the crystallization temperature is typically carried out for a period of time ranging from about 0.5 to about 16 hours, preferably from about 1 to 12 hours, most preferably from about 2 to 9 hours. The temperature may be increased stepwise or continuously. However, continuous heating is preferred.

Crystallisation is continued at the crystallisation temperature until the desired percentage of crystalline material is obtained i.e. from between 5 to 50 % by weight of the amorphous precursor. The crystallisation process can usually last for a period of from several hours to several weeks depending on the desired amount of crystallinity. Effective crystallisation times of from about 2 hours to about 30 days are generally employed with from about 24 to about 240 hours and preferably about 48 hours to about 144 hours, being typically employed. In a specific embodiment, the reaction mixture is kept at the crystallization temperature for a period of from 16 to 96 hours.
The reaction mixture may be kept static or agitated by means of tumbling or stirring of the reaction vessel during hydrothermal treatment. Preferably, the reaction mixture is tumbled or stirred, most preferably stirred. While not essential to the synthesis of the molecular sieve according to the invention, stirring or other moderate agitation of the reaction mixture facilitates the crystallisation procedure.
The product is recovered by any convenient method such as centrifugation or filtration.

The molecular sieve may be used as a catalyst, without further co-formulation, if the particles recovered from the crystallization step are of a size and shape desired for the ultimate catalyst.

**With regards to step (g)**, formulation can be carried out by extrusion and/or pelletising and/or spray-drying in the cases where formulation was not carried out during step (c), after the addition of a required amount of water to the partially or completely crystallized material obtained from step (f). The non-crystalline part of the partially crystallised solid substitutes the matrix and binder in this case. However, optionally the formulating step can be performed in the presence of various other materials, such as Tylose, matrix materials, various inert or catalytically active materials, or various binder materials, to facilitate and to increase the catalyst's resistance yet further to the temperatures and other conditions employed in the organic conversion processes.
Such matrix materials include active and inactive materials and synthetic or naturally occurring zeolites as well as inorganic materials such as alumina, clays, silica and metal oxides. These may also be in the form of gelatinous precipitates, sols, or gels, including mixtures of silica and metal oxides. Various forms of rare earth metals, alumina or alumina sol, titania, xonotlite, zirconia and quartz can also be envisaged. Use of an active material in conjunction with the synthetic molecular sieve, i.e. combined with it, tends to improve the conversion and selectivity of the catalyst in certain organic conversion processes. Frequently, molecular sieve materials have been incorporated into naturally occurring clays, e.g. bentonite and kaolin. These materials, i.e. clays, oxides etc., function, in part, as binders for the catalyst.
Naturally occurring clays which can be composited with the molecular sieve crystals include the montmorillonite and kaolin families which include the subbentonites, and the kaolins commonly known as Dixie, McNamee, Georgia, and Florida clays or others in which the main mineral constituent is halloysite, kaolinite, dickite, nacite, or an auxite. Such clays can be used in the raw state as originally mined or initially subjected to calcination, acid treatment, or chemical modification. Binders useful for compositing with the present crystal also include inorganic oxides, notably alumina or silica.
In addition to the foregoing materials, the molecular sieve produced can be composited with a porous matrix material such as aluminum phosphate, silica-alumina, silica-magnesia, silica-zirconia, silica-thoria, silica-beryllia, silica-titania as well as ternary compositions such as silica-alumina-thoria, silica-alumina-zirconia, silica-alumina-magnesia, and silica-magnesia-zirconia. The relative proportions of finely divided molecular sieve material and inorganic oxide matrix can vary widely, with the crystal content ranging from 1 to 90% by weight and more usually, particularly when the composite is prepared in the form of beads, in the range of 2 to 80% by weight of the composite.

When blended with metalloaluminophosphate molecular sieve materials, the amount of MeAPO of the present invention, which is contained in the final catalyst product ranges from 10 to 90% by weight of the total catalyst, preferably 20 to 70% by weight of the total catalyst.
These materials include compositions such as kaolin and other clays, various forms of rare earth metals, alumina or alumina sol, titania, zirconia, quartz, silica or silica sol, and mixtures thereof. Their effect is to densify the catalysts and increase the strength of the formulated catalyst

**With regards to step (h)**, calcination of molecular sieves is known per se. As a result of the molecular sieve crystallization process, the recovered molecular sieve contains within its pores at least a portion of the template used. In a preferred embodiment, activation is performed in such a manner that the template is removed from the molecular sieve, leaving active catalytic sites with the microporous channels of the molecular sieve open for contact with a feedstock. The activation process is typically accomplished by calcining, or essentially heating the molecular sieve comprising the template at a temperature of from 200 to 800° C, preferably 350°C to 600°C in the presence of an oxygen-containing gas. In some cases, it may be desirable to heat the molecular sieve in an environment having a low oxygen concentration. This type of process can be used for partial or complete removal of the template from the intracrystalline pore system. In some particular cases the final molecular sieves can be subjected to a steaming step at a temperature from 550 to 750°C, more preferably from 600 to 720°C, under an atmosphere containing at least 10% of water.

If during the synthesis alkaline or alkaline earth metals have been used, the molecular sieve might be subjected to an ion-exchange step. Conventionally, ion-exchange is done in aqueous solutions using ammonium salts or inorganic acids.

Once the molecular sieve is made, it can be used as itself as a catalyst. In another embodiment it can be formulated into a catalyst by combining the molecular sieve with other materials that provide even further hardness or possibly catalytic activity to the finished catalyst product. However, as pointed out previously, any remaining amorphous phase of the MeAPO can act as a binder.

The present invention also relates to catalysts consisting of the above MeAPO molecular sieves made by the method of the invention or comprising the above MeAPO molecular sieves made by the method of the invention.

**Uses of the MeAPO molecular sieves** synthesized in accordance with the present method include drying gases and liquids; selective molecular separation based on size and polar properties; their use as ion-exchangers; their use as catalysts in cracking, hydrocracking, disproportionation, alkylation, isomerization, oxidation; their use as chemical carriers; their use in gas chromatography; and their use in the petroleum industry to remove normal paraffins from distillates. More precisely they are useful as catalysts in a variety of processes including cracking of, for example, a naphtha feed to light olefin(s) or higher molecular weight (MW) hydrocarbons to lower MW hydrocarbons; hydrocracking of, for example, heavy petroleum and/or cyclic feedstock; isomerization of, for example, aromatics such as xylene; polymerization of, for example, one or more olefin(s) to produce a oligomer product; dewaxing of, for example, hydrocarbons to remove straight chain paraffins; adsorption of, for example, alkyl aromatic compounds for separating out isomers thereof; oligomerization of, for example, straight and branched chain olefin(s); and the synthesis of monoalkylamines and dialkylamines.

**The MeAPO made by the method of the present invention are particularly suited for the catalytic conversion of oxygen-containing, halogenide-containing or sulphur-containing organic compounds to hydrocarbons.** Accordingly, the present invention also relates to a method for making an olefin product from an oxygen-containing, halogenide-containing or sulphur-containing organic feedstock wherein said feedstock is contacted with the catalyst comprising the molecular sieve of this invention under conditions effective to convert the feedstock to olefin products. In this process a feedstock containing an oxygen-containing, halogenide-containing or sulphur-containing organic compound contacts the above described catalyst in a reaction zone of a reactor at conditions effective to produce light olefins, particularly ethylene and propylene. Typically, the oxygen-containing, halogenide-containing or sulphur-containing organic feedstock is contacted with the catalyst when the oxygen-containing, halogenide-containing or sulphur-containing organic compounds are in the vapour phase. Alternatively, the process may be carried out in a liquid or a mixed vapour/liquid phase. In this process, converting oxygen-containing, halogenide-containing or sulphur-containing organic compounds, olefins can generally be produced at a wide range of temperatures. An effective operating temperature range can be from about 200° C to 700° C. At the lower end of the temperature range, the formation of the desired olefin products may become markedly slow. At the upper end of the temperature range, the process may not form an optimum amount of product. An operating temperature of at least 300° C, and up to 575° C is preferred.

The pressure also may vary over a wide range. Preferred pressures are in the range of about 5 kPa to about 5 MPa, with the most preferred range being of from about 50 kPa to about 0.5 MPa. The foregoing pressures refer to the partial pressure of the oxygen-containing, halogenide-containing, sulphur-containing organic compounds and/or mixtures thereof.
The process can be carried out in any system using a variety of transport beds, although a fixed bed or moving bed system could be used. Advantageously, a fluidized bed is used. It is particularly desirable to operate the reaction process at high space velocities. The process can be conducted in a single reaction zone or a number of reaction zones arranged in series or in parallel. Any standard commercial scale reactor system can be used, for example fixed bed, fluidised or moving bed systems. The commercial scale reactor systems can be operated at a weight hourly space velocity (WHSV) of from 0.1 hr⁻¹ to 1000 hr⁻¹.
One or more inert diluents may be present in the feedstock, for example, in an amount of from 1 to 95 molar percent, based on the total number of moles of all feed and diluent components fed to the reaction zone. Typical diluents include, but are not necessarily limited to helium, argon, nitrogen, carbon monoxide, carbon dioxide, hydrogen, water, paraffins, alkanes (especially methane, ethane, and propane), aromatic compounds, and mixtures thereof. The preferred diluents are water and nitrogen. Water can be injected in either liquid or vapour form.
The oxygenate feedstock is any feedstock containing a molecule or any chemical having at least an oxygen atom and capable, in the presence of the above MeAPO catalyst, to be converted to olefin products. The oxygenate feedstock comprises at least one organic compound which contains at least one oxygen atom, such as aliphatic alcohols, ethers, carbonyl compounds (aldehydes, ketones, carboxylic acids, carbonates, esters and the like). Representative oxygenates include but are not necessarily limited to lower straight and branched chain aliphatic alcohols and their unsaturated counterparts. Examples of suitable oxygenate compounds include, but are not limited to: methanol; ethanol; n-propanol; isopropanol; C₄-C₂₀ alcohols; methyl ethyl ether; dimethyl ether; diethyl ether; di-isopropyl ether; formaldehyde; dimethyl carbonate; dimethyl ketone; acetic acid; and mixtures thereof. Representative oxygenates include lower straight chain or branched aliphatic alcohols, their unsaturated counterparts.
Analogously to these oxygenates, compounds containing sulphur or halides may be used. Examples of suitable compounds include methyl mercaptan; dimethyl sulfide; ethyl mercaptan; di-ethyl sulfide; ethyl monochloride; methyl monochloride, methyl dichloride, n-alkyl halides, n-alkyl sulfides having n-alkyl groups comprising the range of from about 1 to about 10 carbon atoms; and mixtures thereof. Preferred oxygenate compounds are methanol, dimethyl ether, or a mixture thereof.

The method of making the olefin products from an oxygenate feedstock can include the additional step of making the oxygenate feedstock from hydrocarbons such as oil, coal, tar sand, shale, biomass and natural gas. Methods for making oxygen-containing, halogenide-containing, sulphur-containing-containing organic feedstocks are known in the art. These methods include fermentation to alcohol or ether, making synthesis gas, then converting the synthesis gas to alcohol or ether. Synthesis gas can be produced by known processes such as steam reforming, autothermal reforming and partial oxidization in case of gas feedstocks or by reforming or gasification using oxygen and steam in case of solid (coal, organic waste) or liquid feedstocks. Methanol, methylsulfide and methylhalides can be produced by oxidation of methane with the help of dioxygen, sulphur or halides in the corresponding oxygen-containing, halogenide-containing or sulphur-containing organic compound.

**The invention also covers a process for recycling a MeAPO molecular sieve** used to make olefin products from an oxygenate feedstock comprising the steps:
x). contacting said feedstocks with a MeAPO molecular sieve under conditions effective to convert feedstock to olefin products;
y). recovering the spent MeAPO molecular sieve and/or the MeAPO attrition particles (fines) produced during step (x); and
z). carrying out the process of claim 1 for obtaining a MeAlPO molecular sieve catalyst, comprising the steps (a) to (g) wherein the spent MeAPO and/or the MeAPO attrition particles (fines) produced during step (x) is/are used as the first MeAPO molecular sieve.

One skilled in the art will also appreciate that the olefin products made by the oxygenate-to-olefin conversion reaction using the molecular sieve of the present invention can be polymerized to form polyolefins, particularly polyethylenes and polypropylenes.

### [Examples]

### Example 1

A sample of SAPO-34 synthesised according to an adapted example of US 4499327. This sample was calcined 6h at 600°C and showed Si content (Si/(Si+Al+P)) 0.41 and represents cubic crystal morphology with the average size 0.4 µm.

The sample is hereinafter identified as Comparative I.

### Example 2

Amorphous precursor A1 was obtained by co-precipitation of the mixture of Al(NO₃)₃, colloidal silica Ludox LS-30 (30% SiO₂) (registered trademark of E.I. duPont de Nemours and Company) and H₃PO₄ by slow addition of a solution of NH₄OH under stirring to increase the pH of the initial mixture from 1 to about 7 (Table 1). The obtained solid was filtered and washed with distilled water, followed by drying at 110°C and calcinations at 400°C for 3h.

Amorphous precursor A2 was obtained by co-precipitation of the mixture of Al(NO₃)₃, Ludox LS-30® and H₃PO₄ by slow addition of a solution of NH₄OH under stirring to increase the pH of the initial mixture from 1 to about 6 (Table 1) followed by introduction of an amount of SAPO-34 and further pH increase by NH₄OH addition to 7. The obtained solid was filtered and washed with distilled water, followed by drying at 110°C and calcinations at 400°C for 3h. The XRD patterns shown in Fig. 1-2 are similar and confirm the amorphous nature of both samples (precursors). The only difference is a very small impurity of SAPO-34 observed in Fig. 2.

The composition of the solid was similar to the composition of the solution. The samples of the amorphous precursors are hereinafter identified as A1 and A2. The composition of each sample is provided in Table 1.

**Table 1**

| **Precursor** | **A1** | **A2** |
|---|---|---|
| Reagents | | |
| Al(NO₃)₃*9H₂O | 187,5g | 187,5g |
| H₂O | 292 ml | 292 ml |
| H₃PO₄ (85wt%) | 57.2g | 57.2g |
| Ludox LS-30 | 15 | 15 |
| SAPO-34 calc (sample from example 1) | 0 | 3g |

| Composition | | |
|---|---|---|
| Al₂O₃/mol | 1 | 1 |
| P₂O₅/mol | 1 | 1 |
| SiO₂/mol | 0.3 | 0.3 |

### Examples 3-6

Examples 3-6 were prepared according to data presented in Table 2 below. A specified quantity of amorphous precursor was weighed, incipient wetness impregnated by an aqueous solution of template and put into an autoclave. The autoclave was then sealed and placed in an oven under a rotation of 10 rpm. The crystallization was performed during 3 days at 160°C. After crystallization the solid was washed, dried at 110°C for 16h and calcined in air at 600°C for 6h (1°C/min).

Fig. 3-6 present the XRD patterns of the non-calcined samples.

**Table 2**

| **Example** | **3** | | **4** | **5** | **6** |
|---|---|---|---|---|---|
| | **Comparative example II** | | **Working Example** | **Comparative example III** | **Working Example** |
| **Synthesis recipe** | | | | | |
| Precursor | **A1** | | **A2** | **A1** | **A2** |
| Weight of precursors, g | 15 | | 15 | 15 | 15 |
| Template solution 40wt% TEAOH in water | 18.4g | | 18.4g | 25g | 25g |
| **Crystallization conditions** | 3days, 160°C, 10 rpm | | | | |
| TEMP / P₂O₅ molar | 0.9 | 0.9 | | 1.2 | 1.2 |
| | | | | | |
| Results of XRD | amorphous phase | SAPO-34 | | SAPO-34 + traces of amorphous | SAPO-34 |

Example 4 illustrates that the presence of small amount of crystals (3-5 wt%) in the initial precursor facilitates crystallization of the amorphous precursor. The crystallization takes place at milder condition and allows a reduction of at least 25wt% of the required template. Examples 3 and 5 show that the crystallization from the dried precursors requires high template content and even with the ratio TEAOH/P₂O₅ -1.2, traces of amorphous material are still present. Examples 3 and 5 require probably much longer times of crystallization. On the contrary, the addition of a very small amount of around 3-5 wt% of SAPO-34 allows to reduce significantly the amount of template required for crystallization i.e. as shown by examples 4 and 6.

### Example 7

Catalyst tests were performed on 2g catalyst samples with a pure methanol feed at 450°C, WHSV=1.6h⁻¹, P=1.3 bara in a fixed-bed, down flow stainless-steel reactor. Catalyst powders were pressed into wafers and crushed to 35-45 mesh particles. Prior to the catalytic runs all catalysts were heated in flowing N₂ (5 NI/h) to 550°C. This temperature was maintained for 2h and then the reactor was cooled down to the reaction temperature. Analysis of the products was performed on-line by a gas chromatograph equipped with a capillary column.
The catalyst performances are compared at substantially complete MeOH conversion just before breakthrough point (appearance of DME in the effluent). Table 3 presents data on C-basis and coke-free basis.

**Table 3. MTO performance**

| **Sample** | **Example 4** | **Comparative I** |
|---|---|---|
| **Feed** | **MeOH** | **MeOH** |
| **WHSV, h-1** | **1.6 h⁻¹** | **1.6 h⁻¹** |
| **T, °C** | **450** | **450** |

| **Compounds** | | |
|---|---|---|
| **C3-/C2-** | **1,0** | **0,7** |
| **C2-+C3-** | **77,7** | **77,5** |
| **Ethylene (C2-)** | **39,8** | **44,8** |
| **Propylene (C3-)** | **37,9** | **32,7** |

The data presented in table 3 demonstrates a good performance in MTO of the sample synthesized with reduced amount of template. Moreover the sample according to the invention demonstrated a higher propylene yield for the same total C2-+C3- yield.

Thus, this invention proposes a solution to re-using SAPO waste whilst simultaneously decreasing the amount of template required for synthesis of the new SAPO catalyst. In addition the new SAPO catalyst is more propylene efficient than a conventional one.

## Claims

1. A process for obtaining a metalloaluminophosphate (MeAPO) molecular sieve comprising the following steps in the order given:
a) providing an aqueous solution containing sources of at least 2 of the following:
- aluminium (Al),
- phosphorous (P),
- metal (Me);
b) adding a first MeAPO molecular sieve to the solution and modifying the pH before and/or after the addition of the first MeAPO molecular sieve to obtain an amorphous precursor;
c) separating the amorphous precursor from the water, optionally including formulation;
d) optionally washing and drying at a temperature below 450°C of the amorphous precursor;
e) contacting the amorphous precursor with an organic template-containing aqueous solution and with a source of Al, P or Me, which is not already present in step (a), optionally additional sources of Al and/or P and/or Me and optionally in the presence of aliphatic alcohols;
f) performing a crystallization of the amorphous precursor under autogeneous conditions so as to increase the concentration of the crystalline molecular sieve in respect to the initial precursor and so as to obtain a second MeAPO molecular sieve.

2. The process according to claim 1 wherein the first MeAPO molecular sieve is a spent MeAPO molecular sieve.

3. The process according to claim 1 or claim 2 wherein the MeAPO molecular sieve is in the form of attrition particles (fines).

4. The process according to any one of the preceding claims wherein 0.1-50 wt % of the first MeAPO molecular sieve, based on the dry weight of the solution prepared in step a), is added to the solution of step (b).

5. The process according to any one of the preceding claims wherein the solution of step (a) contains sources of Al and P in a molar ratio Al/P ranging from 0.5 to 5.

6. The process according to any one of the preceding claims wherein the precursor is formulated prior to step (c).

7. The process according to any one of claims 1 to 5 comprising a further step (g) wherein the molecular sieve obtained from step (f) is formulated by extrusion or spray-drying, optionally in the presence of a binder, if not formulated during step (c).

8. The process according to any one of the preceding claims comprising a further step (h) wherein the molecular sieve obtained from step (f) or step (g) is calcined, steamed or ion-exchanged.

9. The process according to any one of the preceding claims wherein Me is silicon.

10. MeAPO obtainable by the process according to any one of the preceding claims wherein the structure is essentially CHA, AEI, LEV, ERI, AFI or a mixture thereof.

11. MeAPO according to claim 10 wherein the structure is essentially SAPO-18 or SAPO-34 or a mixture thereof.

12. A catalyst consisting of the MeAPO molecular sieves according to any one of claims 10 or 11 or comprising the MeAPO molecular sieves according to any one of claims 10 or 11.

13. Process for making an olefin product from an oxygenate feedstock wherein said oxygenate feedstock is contacted with the catalyst of claim 12 under conditions effective to convert the oxygenate feedstock to olefin products.

14. Process for making an olefin product from an organic sulphur feedstock wherein said organic sulphur feedstock is contacted with the catalyst of claim 12 under conditions effective to convert the organic sulphur feedstock to olefin products.

15. Process for recycling a MeAPO molecular sieve used to make olefin products from an oxygenate feedstock comprising the following steps:
a). contacting said feedstock with a MeAPO molecular sieve under conditions effective to convert feedstock to olefin products;
b). recovering the spent MeAPO molecular sieve and/or the spent MeAPO fines produced during step (a);
c). carrying out the process of any one of claims 1 to 9 wherein the spent MeAPO and/or fines produced during step (a) is/are used as the first MeAPO molecular sieve to obtain said second MeAPO molecular sieve.
